# EUROPÄISCHE PATENTANMELDUNG

(11) **EP 0 634 398 A1**
(43) Veröffentlichungstag der Anmeldung: **18.01.1995**
(21) Anmeldenummer: 94109058.1
(22) Anmeldetag: 14.06.1994
(51) Int. Cl.: C07D 211/22, C07D 319/18, C07D 405/06, C07D 401/06, C07D 405/14, A61K 31/445, A61K 31/47, A61K 31/55

(54) **4-Phenylpiperidinyl-1/-Piperazinyl-1-Ethyl substituierte benzokondensierte Heterocyclen als antiarrhythmische Wirkstoffe**

(30) Priorität: 26.06.1993 DE 4321366
(71) Anmelder: MERCK PATENT GmbH, D-64293 Darmstadt (DE)
(72) Erfinder: Baumgarth, Manfred, Dr., D-64297 Darmstadt (DE); Lues, Inge, Dr., D-64297 Darmstadt (DE); Minck, Klaus-Otto, Dr., D-64372 Ober-Ramstadt (DE); Beier, Norbert, Dr., D-64354 Reinheim (DE)

(57) **Zusammenfassung**

Piperidin- und Piperazinderivate der Formel I 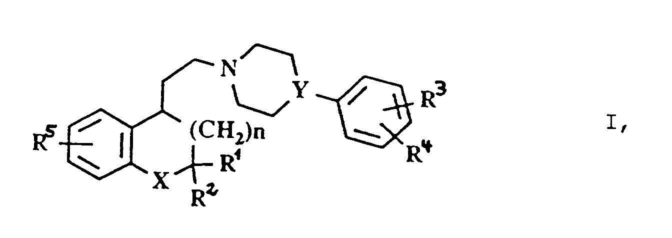
worin
- R¹ und R²: H oder A,
- R³, R⁴ und R⁵: jeweils unabhängig voneinander H, Hal, OH, OA, OAc, NO₂, NH₂, NHAc, NHSO₂A oder CN,
oder
- R³ und R⁴: zusammen auch -O-(CH₂)ₘ-O-,
- n: 0, 1 oder 2
- X: O oder CH₂, wenn n = 0 oder 2, oder CH₂, NH, NA oder NAc, wenn n = 1,
- Y: CH oder N
- m: 1 oder 2
- Hal: F, Cl, Br oder I,
- A: Alkyl mit 1-6 C-Atomen und
- Ac: Alkanoyl mit 1-8 C-Atomen, Aralkanoyl mit 1-10 C-Atomen oder Aroyl mit 7-11 C-Atomen
bedeuten,
sowie deren physiologisch unbedenkliche Salze zeigen antiarrhythmische Wirkungen.

## Beschreibung

Die Erfindung betrifft neue Piperidin- und Piperazinderivate der Formel I 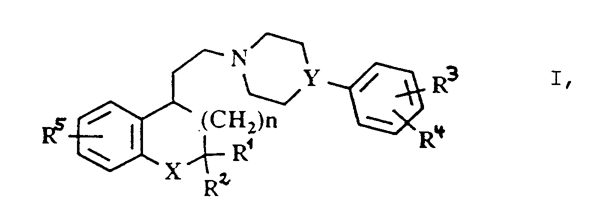
worin
- R¹ und R²: H oder A,
- R³, R⁴ und R⁵: jeweils unabhängig voneinander H, Hal, OH, OA, OAc, NO₂, NH₂, NHAc, NHSO₂A oder CN,
oder
- R³ und R⁴: zusammen auch -O-(CH₂)m-O-,
- n: 0, 1 oder 2
- X: O oder CH₂, wenn n = 0 oder 2, oder CH₂, NH, NA oder NAc, wenn n = 1,
- Y: CH oder N
- m: 1 oder 2
- Hal: F, Cl, Br oder I,
- A: Alkyl mit 1-6 C-Atomen
und
- Ac: Alkanoyl mit 1-8 C-Atomen, Aralkanoyl mit 1-10 C-Atomen oder Aroyl mit 7-11 C-Atomen
bedeuten,
sowie deren physiologisch unbedenkliche Salze.

Der Erfindung lag die Aufgabe zugrunde, neue Verbindungen aufzufinden, die zur Herstellung von Arzneimitteln verwendet werden können.

Es wurde gefunden, daß die Verbindungen der Formel I und ihre physiologisch unbedenklichen Säureadditionssalze bei guter Verträglichkeit wertvolle pharmakologische Eigenschaften besitzen. So zeigen sie insbesondere antiarrhythmische bzw. die Refraktärzeit des Herzens verlängernde, positiv inotrope Wirkungen.

Die Herzwirkung kann z.B. an narkotisierten oder wachen Ratten, Meerschweinchen, Hunden, Katzen, Affen oder Minischweinen, die positiv inotrope Wirkung auch an isolierten Herzpräparationen (z.B. Vorhof, Papillarmuskel oder perfundiertes Ganzherz) von Ratte, Meerschweinchen, Katze oder Hund ermittelt werden, z.B. nach Methoden, wie sie in Arzneimittelforschung, Band 31 (I) Nr. 1a (1981), Seiten 141 bis 170, oder von Schliep et al. im 9th International Congress of Pharmacol., London (1984), Abstracts of papers 9P, beschrieben sind.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe dienen.

Gegenstand der Erfindung sind dementsprechend die Verbindungen der Formel I, ihre Säureadditionssalze sowie ein Verfahren zu ihrer Herstellung, dadurch gekennzeichnet, daß man eine Verbindung der Formel II 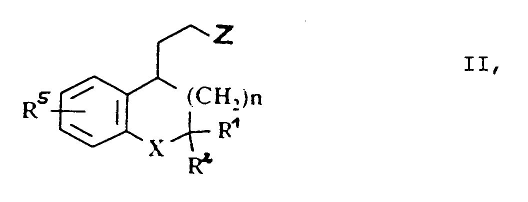
worin
- Z: Cl, Br, OH oder eine reaktionsfähige, funktionell abgewandelte OH-Gruppe bedeutet und
- R¹, R², R⁵, X und n: die angegebenen Bedeutungen haben,
mit einer Verbindung der Formel III 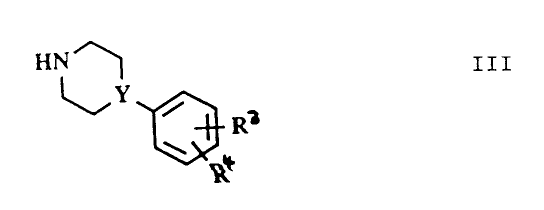
worin
- R³, R⁴ sowie Y: die angegebenen Bedeutungen haben,
umsetzt
oder daß man eine Verbindung, die an sich der Formel I entspricht, aber anstelle einer oder mehrerer CH₂-Gruppen eine oder mehrere reduzierbare Gruppen besitzt, durch Reduktion mit einem geeigneten Reduktionsmittel in eine Verbindung der Formel I überführt und/oder daß man in einer Verbindung der Formel I einen oder mehrere der Reste R³, R⁴ und/oder R⁵ bzw. X in andere Reste R³, R⁴ und/oder R⁵ bzw. X umgewandelt und/oder daß man eine basische Verbindung der Formel I durch Behandeln mit einer Säure in eines ihrer physiologisch unbedenklichen Säureadditionssalze umwandelt.

Vor- und nachstehend haben R¹, R², R³, R⁴, R⁵, n, m, X, Y, Z, Hal, A und Ac die bei den Formeln I, II und III angegebenen Bedeutungen, wenn nicht ausdrücklich etwas anderes angegeben ist.

Der Rest A bedeutet Alkyl mit 1, 2, 3, 4, 5 oder 6, insbesondere 1, 2 oder 3 C-Atomen, vorzugsweise Methyl, ferner auch Ethyl, n-Propyl, Isopropyl, n-Butyl, Isobutyl, sek.-Butyl oder tert.-Butyl. OA ist vorzugsweise Methoxy, ferner auch Ethoxy, n-Propoxy, Isopropoxy, n-Butoxy, Isobutoxy, sek.-Butoxy oder tert.-Butoxy. In -NA- oder NHSO₂A ist A vorzugsweise Methyl.

Die Gruppe Ac steht vorzugsweise für Alkanoyl mit 1-8 C-Atomen, insbesondere mit 1, 2, 3, 4 oder 5 C-Atomen; im einzelnen bevorzugt Acetyl, ferner bevorzugt Formyl, Propionyl, Butyryl, Isobutyryl, Valeryl, Isovaleryl, Pivaloyl (Trimethylacetyl), weiterhin bevorzugt gegebenenfalls substituiertes Aroyl mit 7-15 C-Atomen, wobei als Substituenten insbesondere 1-3, vorzugsweise eine der folgenden Gruppen in Frage kommen: Alkyl, Alkoxy, Alkylthio, Alkylsulfinyl oder Alkylsulfonyl mit jeweils 1-3, vorzugsweise 1 oder 2 C-Atomen, Methylendioxy, ferner OH, F, Cl, Br, I, NO₂, NH₂, Alkylamino oder Dialkylamino mit jeweils 1-3, vorzugsweise 1 oder 2 C-Atomen in der Alkylgruppe. Einzelne bevorzugte Aroylreste sind Benzoyl, o-, m- oder p-Toluyl, o-, m- oder p-Methoxybenzoyl, 2,3-, 2,4-, 2,5-, 2,6-, 3,4- oder 3,5-Dimethoxybenzoyl, 2,3,4-, 2,3,5-, 2,3,6-, 2,4,5-, 2,4,6- oder 3,4,5-Trimethoxybenzoyl, o-, m- oder p-Methylthiobenzoyl, o-, m- oder p-Methylsulfinylbenzoyl, o-, m- oder p-Methylsulfonylbenzoyl, 2,3- oder 3,4-Methylendioxybenzoyl, 1- oder 2-Naphthoyl. Ac kann weiterhin für Aralkanoyl mit 1-10 C-Atomen wie z.B. Phenylacetyl, 2- oder 3-Phenylpropionyl oder 2-, 3- oder 4-Phenylbutyryl stehen.

Falls eine Verbindungen der Formel I, II oder III mehrere Gruppen A und/oder Ac enthält, so können diese gleich oder voneinander verschieden sein.

Die Reste R¹ und R² bedeuten vorzugsweise jeweils Wasserstoff oder Methyl.

R³, R⁴ und R⁵ stehen jeweils vorzugsweise für Wasserstoff oder Methoxy; R⁵ auch für CN, während R³ und R⁴ zusammen auch bevorzugt Methylendioxy oder Ethylendioxy bedeuten können.

Die Bedeutung von X ist Sauerstoff oder -CH₂-, wenn n 0 oder 2 bedeutet. Sofern n = 1 ist, bedeutet X jedoch -CH₂-, -NH-, -NA- oder -NAc-, wobei A und Ac die zuvor angegebenen Bedeutungen haben. Y bedeutet N, vorzugsweise jedoch CH.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat.

Einige bevorzugte Gruppen von Verbindungen können durch die folgenden Teilformeln Ia bis Ii ausgedrückt werden, die der Formel I entsprechen und worin die nicht näher bezeichneten Reste und Parameter die bei der Formel I angegebene Bedeutung haben, worin jedoch
- in Ia: R¹, R² und R⁵ Wasserstoff und R³ und R⁴ jeweils Methoxy bedeuten;
- in Ib: R¹, R² und R⁵ Wasserstoff und R³ und R⁴ zusammen Methylen- oder Ethylendioxy bedeuten;
- in Ic: R¹, R² und R⁵ Wasserstoff, n 0, 1 oder 2 und X -CH₂- bedeuten;
- in Id: R¹, R² und R⁵ Wasserstoff, n 0 oder 2 und X Sauerstoff bedeuten;
- in Ie: R¹, R² und R⁵ Wasserstoff, n = 1 und X NH bedeuten;
- in If: R¹, R² und R⁵ jeweils Wasserstoff, R³ und R⁴ Methoxy, X -CH₂- und Y CH bedeuten;
- in Ig: R¹, R² und R⁵ Wasserstoff, R³ und R⁴ jeweils Methoxy, X -CH₂- und Y Stickstoff bedeuten;
- in Ih: R¹, R² und R⁵ Wasserstoff, R³ und R⁴ zusammen Ethylendioxy, X -CH₂- und Y Stickstoff bedeuten;
- in Ii: R¹ und R² jeweils Methyl, R⁵ Wasserstoff und R³ und R⁴ jeweils Methoxy bedeuten:

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

Die Ausgangsstoffe der Formel II und III sind teilweise bekannt. Sofern sie nicht bekannt sind, können sie nach an sich bekannten Methoden hergestellt werden. Die Herstellung der Verbindungen der Formel II erfolgt beispielsweise ausgehend von Indan-1-on, 6,7,8,9-Tetrahydro-5H-benzocyclohepten-5-on, 1,2,3,4-Tetrahydronaphthalin-1-on, 2,3-Dihydrobenzofuran-3-on, 2,3,4,5-Tetrahydro-1-benzoxepin-5-on oder 1,2,3,4-Tetrahydro-chinolin-4-on bzw. ihren substituierten Derivaten durch Umsetzung mit Diethylethoxycarbonylmethanphosphonat (Wittig-Horner Reaktion; Org. Reactions 25, 73 (1977)), anschließende Reduktion mit H₂/Pd-C, Reduktion des entstehenden Produktes mit Diboran und gegebenenfalls weitere Aktivierung des Restes Z durch Umwandlung in einen anderen Rest Z nach an sich bekannten, hier nicht näher erwähnten Methoden.

Die Verbindungen der Formel III können beispielsweise durch Umsetzung von Di-(2-chlorethyl)-amin mit Anilin oder einem entsprechenden, am Phenylring substituierten Derivat des Anilins erhalten werden. Die Piperidine der Formel III sind z.B. durch Reaktion von NH₃ mit 1,5-Dichlor-3-phenyl-pentan oder mit homologen am Phenylring substituierten Verbindungen zugänglich.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Ferner ist es möglich, eine Verbindung der Formel I zu erhalten, indem man eine Verbindung die an sich der Formel I entspricht, aber anstelle einer oder mehrerer CH₂-Gruppen, eine oder mehrere reduzierbare Gruppen enthält, reduziert, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen enthalten, reduktiv in eine Verbindung der Formel I zu überführen. Vorzugsweise bedient man sich hierzu des nascierenden Wasserstoffs oder komplexer Metallhydride, ferner der Reduktion nach Wolff-Kishner sowie der Reduktionen mit Wasserstoffgas unter Übergangsmetallkatalyse.

Bevorzugte Ausgangsstoffe für die Reduktion entsprechen der Formel IV 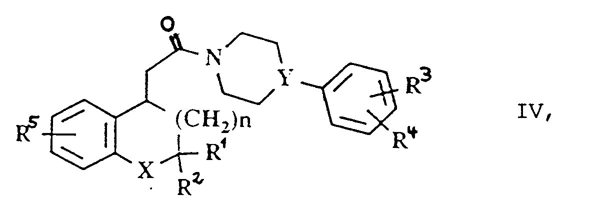
worin
R¹, R², R³, R⁴, R⁵, n, X und Y die zuvor bei der Formel I angegebenen Bedeutungen haben.

Verbindungen der Formel IV sind herstellbar durch Umsetzung von Verbindungen, die an sich der Formel II entsprechen, aber anstelle der -CH₂-Z-Gruppe eine -COCl- oder -COBr-Gruppe aufweisen, mit Piperidinen oder Piperazinen der Formel III.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerig-alkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie LiAlH₄, NaBH₄, Diisobutylaluminiumhydrid oder NaAl (OCH₂CH₂OCH₃)₂H₂ sowie Diboran eingesetzt werden, falls erwünscht unter Zusatz von Katalysatoren wie BF₃, AlCl₃ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit NaBH₄ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich die -CO-Gruppen in den Säureamiden mit LiAlH₄ in THF bei Temperaturen zwischen etwa 0 und 66° zu CH₂-Gruppen reduzieren.

Darüber hinaus ist es möglich, bestimmte Reduktionen durch Verwendung von H₂-Gas unter katalytischer Wirkung von Übergangsmetallen, wie z.B. Raney-Ni oder Pd durchzuführen. Man kann auf diese Weise z.B. Cl,Br, I, SH oder in bestimmten Fällen auch OH-Gruppen durch Wasserstoff ersetzen. Ebenso können Nitrogruppen durch katalytische Hydrierung mit Pd/H₂ in Methanol in NH₂-Gruppen umgewandelt werden.

Weiterhin kann man eine Verbindung der Formel I nach an sich bekannten Methoden in eine andere Verbindung der Formel I umwandeln.

Ether der Formel I, in denen beispielsweise R³, R⁴ und/oder R⁵ für eine OA-Gruppe stehen, können auf an sich bekannte Weise gespalten werden, wobei die entsprechenden Hydroxyderivate entstehen. Z.B. kann man die Ether spalten durch Behandeln mit Dimethylsulfid-Bortribromid-Komplex, z.B. in Toluol, Ethern wie THF oder Dimethylsulfoxid, oder durch Verschmelzen mit Pyridin- oder Anilin-hydrohalogeniden, vorzugsweise Pyridinhydrochlorid, bei etwa 150-250°.

Die Phenylringe der Verbindungen der Formel I können, sofern Nebenreaktionen auszuschließen sind, unter den Bedingungen der Friedel-Crafts-Reaktionen chloriert, bromiert oder alkyliert werden, indem man das entsprechende Halogen oder Alkylchlorid bzw. Alkylbromid unter Katalyse von Lewis-Säuren, wie z.B. AlCl₃, FeBr₃ oder Fe, bei Temperaturen zwischen 30° und 150°, zweckmäßig zwischen 50° und 150° in einem inerten Lösungsmittel, wie z.B. Kohlenwasserstoffen, THF oder Tetrachlorkohlenstoff mit der zu derivatisierenden Verbindung der Formel I umsetzt.

Ferner ist es möglich, daß man eine Verbindung der Formel I, in der X = NH ist, durch Alkylierung oder Acylierung, nach Methoden wie sie für Amine allgemein üblich und bekannt sind, in entsprechende Verbindungen der Formel I, in denen X = NA oder NAc ist, umwandelt.

Die Verbindungen der Formel I können ein oder zwei Asymmetriezentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei Asymmetriezentren auf, dann fallen sie bei der Synthese im allgemeinen als Gemische von Racematen an, aus denen man die einzelnen Racemate, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet.

Als Trennmittel eignen sich z.B. optisch aktive Säuren, wie die D- und L-Formen von Weinsäuren, Dibenzoylweinsäure, Diacetylweinsäure, Camphersulfonsäuren, Mandelsäure, Äpfelsäure oder Milchsäure. Die verschidenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Eine erhaltene Base der Formel I kann mit einer Säure in das zugehörige Säureadditionssalz überführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure,Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über freie acide Gruppen verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoffe(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlenhydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden. Sie eignen sich insbesondere zur Behandlung von Arrhythmien und von Tachykardien.

Dabei werden die erfindungsgemäßen Substanzen in der Regel in Analogie zu bekannten antiarryhthmisch wirksamen Substanzen wie Aprindin, Flecainid oder Amiodaron, vorzugsweise in Dosierungen zwischen etwa 1 und 100 mg, insbesondere zwischen 2 und 20 mg pro Dosierungseinheit verabreicht.

Die tägliche Dosierung liegt vorzugsweise zwischen etwa 0,02 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":
Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatographie an Kieselgel und/oder Kristallisation.

Vor- und nachstehend sind alle Temperaturen in Celsiusgraden angegeben.

### Beispiel 1

Man löst 5,4 g 2-(6,7,8,9-Tetrahydro-5H-benzocyclohepten-5-yl)-ethylbromid [erhältlich durch Umsetzung von 6,7,8,9-Tetrahydro-5H-benzocyclohepten-5-on mit Diethylethoxycarbonylmethanphosphonat, anschließende Hydrierung mit H₂/Pd-C, Reduktion mit BH₃ x THF zum Alkohol und anschließende Substitution zum Bromid] 6,7 g 4-(3,4-Dimethoxyphenyl)-piperidinhydrochlorid, 5,8 g K₂CO₃ und 3,6 g KI in 160 ml Ethylmethylketon und kocht 3 Stunden. Nach üblicher Aufarbeitung erhält man 1-(2-(6,7,8,9-Tetrahydro-5H-benzocyclohepten-5-yl)-ethyl)-4-(3,4-dimethoxyphenyl)-piperidin.

Nachfolgende Umsetzung mit Fumarsäure liefert nach Kristallisation das entsprechende Fumarat, F. 127-128°.

Analog erhält man durch Umsetzung von 4-(3,4-Dimethoxyphenyl)-piperidin-hydrochlorid
mit 2-(2,3,4,5-Tetrahydro-1-benzoxepin-5-yl)-ethylbromid: 1-(2-(2,3,4,5-Tetrahydro-1-benzoxepin-5-yl)-ethyl)-4-(3,4-dimethoxyphenyl)-piperidin, Fumarat, F. 163°;
mit 2-(2,3,4,5-Tetrahydro-6-amino-1-benzoxepin-5-yl)-ethylbromid: 1-(2-(2,3,4,5-Tetrahydro-6-amino-1-benzoxepin-5-yl)-ethyl)-4-(3,4-dimethoxyphenyl)-piperidin;
mit 2-(2,3,4,5-Tetrahydro-6-acetylamino-1-benzoxepin-5-yl)-ethylbromid: 1-(2-(2,3,4,5-Tetrahydro-6-acetylamino-1-benzoxepin-5-yl)-ethyl)-4-(3,4-dimethoxyphenyl)-piperidin;
mit 2-(2,3,4,5-Tetrahydro-6-methoxy-1-benzoxepin-5-yl)-ethylbromid: 1-(2-(2,3,4,5-Tetrahydro-6-methoxy-1-benzoxepin-5-yl)-ethyl)-4-(3,4-dimethoxyphenyl)-piperidin;
mit 2-(2,3,4,5-Tetrahydro-7-chlor-1-benzoxepin-5-yl)-ethylbromid: 1-(2-(2,3,4,5-Tetrahydro-7-chlor-1-benzoxepin-5-yl)-ethyl)-4-(3,4-dimethoxyphenyl)-piperidin;
mit 2-(2,3,4,5-Tetrahydro-6-brom-1-benzoxepin-5-yl)-ethylbromid: 1-(2-(2,3,4,5-Tetrahydro-6-brom-1-benzoxepin-5-yl)-ethyl)-4-(3,4-dimethoxyphenyl)-piperidin;
mit 2-(2,3,4,5-Tetrahydro-6-chlor-1-benzoxepin-5-yl)-ethylbromid: 1-(2-(2,3,4,5-Tetrahydro-6-chlor-1-benzoxepin-5-yl)-ethyl)-4-(3,4-dimethoxyphenyl)-piperidin;
mit 2-(2,3,4,5-Tetrahydro-7-hydroxy-1-benzoxepin-5-yl)-ethylbromid: 1-(2-(2,3,4,5-Tetrahydro-7-hydroxy-1-benzoxepin-5-yl)-ethyl)-4-(3,4-dimethoxyphenyl)-piperidin;
mit 2-(2,3,4,5-Tetrahydro-7-amino-1-benzoxepin-5-yl)-ethylbromid: 1-(2-(2,3,4,5-Tetrahydro-7-amino-1-benzoxepin-5-yl)-ethyl)-4-(3,4-dimethoxyphenyl)-piperidin;
mit 2-(2,3,4,5-Tetrahydro-6-acetoxy-1-benzoxepin-5-yl)-ethylbromid: 1-(2-(2,3,4,5-Tetrahydro-6-acetoxy-1-benzoxepin-5-yl)-ethyl)-4-(3,4-dimethoxyphenyl)-piperidin;
mit 2-(2,3,4,5-Tetrahydro-7-methoxy-1-benzoxepin-5-yl)-ethylbromid: 1-(2-(2,3,4,5-Tetrahydro-7-methoxy-1-benzoxepin-5-yl)-ethyl)-4-(3,4-dimethoxyphenyl)-piperidin.

### Beispiel 2

Eine Suspension von 0,6 g Lithiumalanat in 35 ml THF wird unter Rühren bei inerten Reaktionsbedingungen mit einer Lösung von 6,3 g 1-(Indan-1-yl-acetyl)-4-(3,4-dimethoxyphenyl)-piperidin [erhältlich durch Umsetzung von 1-Indanon mit Diethylethoxycarbonyl-methanphosphonat, anschließende Hydrierung mit H₂/Pd-C, Überführung des vorliegenden Esters in ein Säurechlorid und Amidbildung mit 4-(3,4-Dimethoxyphenyl)-Piperidin] in 70 ml THF tropfenweise versetzt und 2 Stunden gekocht. Anschließend fügt man weitere 0,8 g Lithiumalanat hinzu und refluxiert weitere 3 Stunden. Die Reaktionsmischung wird danach mit Methanol und anschließend mit Wasser versetzt und wie üblich aufgearbeitet. Nach Chromatographie (Methyl-tert.-butylether/Petrolether/Diethylamin) erhält man 1-(2-(Indan-1-yl)-ethyl)-4-(3,4-dimethoxyphenyl)-piperidin.

Nachfolgende Umsetzung mit Fumarsäure liefert nach Kristallisation das entsprechende Fumarat, F. 156-157°.

Analog erhält man durch Reduktion mit Lithiumaluminiumhydrid und nachfolgende Salzbildung
aus 1-(Tetralin-1-yl-acetyl)-4-(3,4-dimethoxyphenyl)-piperidin: 1-(2-(Tetralin-1-yl)-ethyl)-4-(3,4-dimethoxyphenyl)-piperidin, Fumarat, F. 154-156°;
aus 6-[4-(Tetralin-1-yl-acetyl)-piperazino]-1,4-benzodioxan:
6-[4-(2-(Tetralin-1-yl)-ethyl)-piperazino]-1,4-benzodioxan, Fumarat, F. 160-161°;
aus 1-(2,3-Dihydrobenzofuran-3-yl-acetyl)-4-(3,4-dimethoxyphenyl)-piperidin:
1-(2-(2,3-Dihydrobenzofuran-3-yl)-ethyl)-4-(3,4-dimethoxyphenyl)-piperidin, Fumarat, F. 149-150°.

Analog erhält man durch Reduktion mit Lithiumaluminiumhydrid
aus 1-(6-Methoxy-tetralin-1-yl-acetyl)-4-(3,4-dimethoxyphenyl)-piperidin:
1-(2-(6-Methoxy-tetralin-1-yl)-ethyl)-4-(3,4-dimethoxyphenyl)-piperidin;
aus 6-[4-(6-Amino-tetralin-1-yl-acetyl)-piperazino]-1,4-benzodioxan:
6-[4-(2-(6-Amino-tetralin-1-yl)-ethyl)-piperazino]-1,4-benzodioxan;
aus 1-(1,2,3,4-Tetrahydro-6-chlor-chinolin-4-yl-acetyl)-4-(3,4-dimethoxyphenyl)-piperidin:
1-(2-(1,2,3,4-Tetrahydro-6-chlor-chinolin-4-yl)-ethyl)-4-(3,4-dimethoxyphenyl)-piperidin;
aus 1-(2,3-Dihydro-5-methoxy-benzofuran-3-yl-acetyl)-4-(3,4-dimethoxyphenyl)-piperidin:
1-(2-(2,3-Dihydro-5-methoxy-benzofuran-3-yl)-ethyl)-4-(3,4-dimethoxyphenyl)-piperidin;
aus 1-(Tetralin-1-yl-acetyl)-4-(3,4-dichlorphenyl)-piperidin:
1-(2-(Tetralin-1-yl)-ethyl)-4-(3,4-dichlorphenyl)-piperidin;
aus 6-[4-(6-Brom-tetralin-1-yl-acetyl)-piperazino]-1,4-benzodioxan:
6-[4-(2-(6-Brom-tetralin-1-yl)-ethyl)-piperazino]-1,4-benzodioxan;
aus 1-(1,2,3,4-Tetrahydro-7-methoxy-chinolin-4-yl-acetyl)-4-(3,4-dimethoxyphenyl)-piperidin:
1-(2-(1,2,3,4-Tetrahydro-7-methoxy-chinolin-4-yl)-ethyl)-4-(3,4-dimethoxyphenyl)-piperidin;
aus 1-(2,3-Dihydrobenzofuran-3-yl-acetyl)-4-(3,4-dichlorphenyl)-piperidin:
1-(2-(2,3-Dihydrobenzofuran-3-yl)-ethyl)-4-(3,4-dichlorphenyl)-piperidin.

### Beispiel 3

Eine Lösung von 2,5 g 1-(2-(1,2,3,4-Tetrahydrochinolin-4-yl)-ethyl)-4-(3,4-dimethoxyphenyl)-piperidin (Dihydrochlorid, F. 235-236°) in 35 ml THF wird mit einer Lösung von 0,9 g Acetylchlorid in 10 ml THF versetzt, 2 Stunden bei 50° gerührt, eingedampft und wie üblich aufgearbeitet. Man erhält 1-(2-(1,2,3,4-Tetrahydro-1-acetyl-chinolin-4-yl)-ethyl)-4-(3,4-dimethoxyphenyl)-piperidin.

Analog erhält man durch Acetylierung oder Alkylierung der entsprechenden Tetrahydrochinolinderivate die nachstehenden Verbindungen:
1-(2-(1,2,3,4-Tetrahydro-1-methyl-chinolin-4-yl)-ethyl)-4-(3,4-dimethoxyphenyl)-piperidin;
1-(2-(1,2,3,4-Tetrahydro-1-isopropyl-chinolin-4-yl)-ethyl)-4-(3,4-dimethoxyphenyl)-piperidin;
1-(2-(1,2,3,4-Tetrahydro-1-propionyl-chinolin-4-yl)-ethyl)-4-(3,4-dimethoxyphenyl)-piperidin;
1-(2-(1,2,3,4-Tetrahydro-1-ethyl-chinolin-4-yl)-ethyl)-4-(3,4-dimethoxyphenyl)-piperidin;
1-(2-(1,2,3,4-Tetrahydro-1-methyl-chinolin-4-yl)-ethyl)-4-(3,4-dichlorphenyl)-piperidin;
1-(2-(1,2,3,4-Tetrahydro-1-isopropyl-chinolin-4-yl)-ethyl)-4-(3,4-methylendioxyphenyl)-piperidin;
1-(2-(1,2,3,4-Tetrahydro-1-propionyl-chinolin-4-yl)-ethyl)-4-(3,4-methylendioxyphenyl)-piperidin;
1-(2-(1,2,3,4-Tetrahydro-1-acetyl-chinolin-4-yl)-ethyl)-4-(3,4-methylendioxyphenyl)-piperidin;
1-(2-(1,2,3,4-Tetrahydro-1-acetyl-chinolin-4-yl)-ethyl)-4-(4-methoxyphenyl)-piperidin;
1-(2-(1,2,3,4-Tetrahydro-1-isopropyl-chinolin-4-yl)-ethyl)-4-(4-chlorphenyl)-piperidin;
1-(2-(1,2,3,4-Tetrahydro-1-propionyl-chinolin-4-yl)-ethyl)-4-(4-methoxyphenyl)-piperidin;
1-(2-(1,2,3,4-Tetrahydro-1-propyl-chinolin-4-yl)-ethyl)-4-(4-nitrophenyl)-piperidin;
1-(2-(1,2,3,4-Tetrahydro-1-propionyl-chinolin-4-yl)-ethyl)-4-(4-chlorphenyl)-piperidin;
1-(2-(1,2,3,4-Tetrahydro-1-isopropyl-chinolin-4-yl)-ethyl)-4-phenyl-piperidin;
1-(2-(1,2,3,4-Tetrahydro-1-propionyl-chinolin-4-yl)-ethyl)-4-phenyl-piperidin;
1-(2-(1,2,3,4-Tetrahydro-1-ethyl-chinolin-4-yl)-ethyl)-4-(4-methoxyphenyl)-piperidin.

### Beispiel 4

Ein Gemisch von 4,1 g 1-(2-(Tetralin-1-yl)-ethyl)-4-(3,4-dimethoxyphenyl)-piperidin (Fumarat, F. 154-156°) 3,2 g Pyridinhydrochlorid und 80 ml Pyridin wird 3 Stunden gekocht. Man kühlt ab, dampft ein, arbeitet wie üblich auf und erhält 1-(2-(Tetralin-1-yl)-ethyl)-4-(3,4-dihydroxyphenyl)-piperidin.

### Beispiel 5

Man löst 3,6 g 2-(2,3,4,5-Tetrahydro-7-cyan-1-benzoxepin-5-yl)-ethylbromid [erhältlich ausgehend von 2,3,4,5-Tetrahydro-7-cyan-1-benzoexpin-5-on durch Umsetzung mit Triphenylethylphosphiniumbromid, anschließende Bromierung des Produktes in Allylposition und Reduktion der isolierten Doppelbindung mit Diisobutylaluminiumhydrid (DIBAH)], 1 Äquivalent 4-(3,4-Dimethoxyphenyl)-piperidinhydrochlorid, 3,8 g K₂CO₃ und 2,1 g KI in 120 ml Ethylmethylketon und kocht 3 Stunden. Nach üblicher Aufarbeitung erhält man 1-(2-(2,3,4,5,-Tetrahydro-7-cyan-1-benzoxepin-5-yl)-ethyl)-4-(3,4-dimethoxyphenyl)-piperidin.

Analog erhält man durch Umsetzung von 4-(3,4-Dimethoxyphenyl)-piperidin-hydrochlorid
mit 2-(2,3,4,5-Tetrahydro-6-cyan-1-benzoxepin-5-yl)-ethylbromid:
1-(2-(2,3,4,5-Tetrahydro-6-cyan-1-benzoxepin-5-yl)-ethyl)-4-(3,4-dimethoxyphenyl)-piperidin;
mit 2-(2,3,4,5-Tetrahydro-6-nitro-1-benzoxepin-5-yl)-ethylbromid):
1-(2-(2,3,4,5-Tetrahydro-6-nitro-1-benzoxepin-5-yl)-ethyl)-4-(3,4-dimethoxyphenyl)-piperidin;
mit 2-(2,3,4,5-Tetrahydro-7-cyan-1-benzoxepin-5-yl)-ethylbromid):
1-(2-(2,3,4,5-Tetrahydro-7-cyan-1-benzoxepin-5-yl)-ethyl)-4-(3,4-dimethoxyphenyl)-piperidin;
mit 2-(2,3,4,5-Tetrahydro-8-cyan-1-benzoxepin-5-yl)-ethylbromid):
1-(2-(2,3,4,5-Tetrahydro-8-cyan-1-benzoxepin-5-yl)-ethyl)-4-(3,4-dimethoxyphenyl)-piperidin;
mit 2-(2,3,4,5-Tetrahydro-8-nitro-1-benzoxepin-5-yl)-ethylbromid):
1-(2-(2,3,4,5-Tetrahydro-8-nitro-1-benzoxepin-5-yl)-ethyl)-4-(3,4-di-methoxyphenyl)-piperidin;

### Beispiel 6

Analog Beispiel 5 erhält man durch Umsetzung von 4-(3,4-Dimethoxyphenyl)-piperidin-hydrochlorid mit 2-(6-Nitrotetralin-1-yl)-ethylbromid das 1-(2-(6-Nitrotetralin-1-yl)-ethyl)-4-(3,4-dimethoxyphenyl)-piperidin.

### Beispiel 7

Eine Suspension von 3,2 g 1-(2-(6-Nitrotetralin-1-yl)-ethyl)-4-(3,4-dimethoxyphenyl)-piperidin in 50 ml Methanol wird in Gegenwart von 1,6 g Pd/C (5%ig) bis zum Stillstand der Wasserstoffaufnahme hydriert. Man filtriert den Katalysator ab, arbeitet anschließend wie üblich auf und erhält 1-(2-(6-Aminotetralin-1-yl)-ethyl)-4-(3,4-dimethoxyphenyl)-piperidin.

### Beispiel 8

Analog Beispiel 2 erhält man durch Reduktion von 1-(1,2,3,4-Tetrahydrochinolin-4-yl-acetyl)-4-(3,4-dimethoxyphenyl)-piperidin [erhältlich durch Umsetzung von 1,2,3,4-Tetrahydrochinolin-4-on mit Diethylethoxycarbonylmethanphosphonat, anschließende Hydrierung mit H₂/Pd-C, Verseifung des vorliegenden Esters und anschließende Amidbildung mit 4-(3,4-Dimethoxyphenyl)-piperidin] mit Lithiumaluminiumhydrid 1-(2-(1,2,3,4-Tetrahydrochinolin-4-yl)-ethyl)-4-(3,4-dimethoxyphenyl)-piperidin, F. 235-236° (Dihydrochlorid).

Die nachstehenden Beispiele betreffen pharmazeutische Zubereitungen.

### Beispiel A: Injektionsgläser

Eine Lösung von 100 g eines Wirkstoffes der Formel I und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ × 2 H₂O, 28,48 g Na₂HPO₄ × 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Piperidin- und Piperazin-Derivate der Formel I 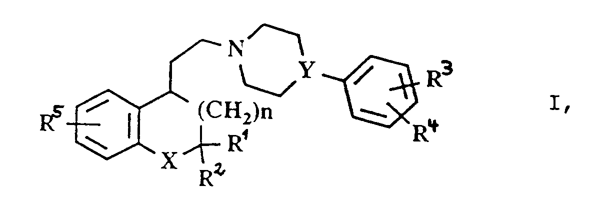 worin
R¹ und R² H oder A,
R³, R⁴ und R⁵ jeweils unabhängig voneinander H, Hal, OH, OA, OAc, NO₂, NH₂, NHAc, NHSO₂A oder CN,
oder
R³ und R⁴ zusammen auch -O-(CH₂)ₘ-O-,
n 0, 1 oder 2
X O oder CH₂, wenn n = 0 oder 2, oder CH₂, NH, NA oder NAc, wenn n = 1,
Y CH oder N
m 1 oder 2
Hal F, Cl, Br oder I,
A Alkyl mit 1-6 C-Atomen
und
Ac Alkanoyl mit 1-8 C-Atomen, Aralkanoyl mit 1-10 C-Atomen oder Aroyl mit 7-11 C-Atomen
bedeuten,
sowie deren physiologisch unbedenkliche Salze.

2. Ein Enantiomer einer Verbindung der Formel I gemäß Anspruch 1.

3. (a) 1-[2-(6,7,8,9-Tetrahydro-5H-benzocyclohepten-5-yl)-ethyl]-4-(3,4-dimethoxyphenyl)-piperidin;
(b) 5-[2-(4-(3,4-Dimethoxyphenyl)-1-piperidinyl)-ethyl]-2,3,4,5-tetrahydro-1-benzoxepin;
(c) 3-[2-(4-(3,4-Dimethoxyphenyl)-1-piperidinyl)-ethyl]-2,3-dihydrobenzofuran;
(d) 1-[2-(Tetralin-1-yl)-ethyl]-4-(3,4-dimethoxy-phenyl)-piperidin.
(e) 1-[2-(Indan-1-yl)-ethyl]-4-(3,4-dimethoxy-phenyl)-piperidin;
(f) 1-[2-(1,2,3,4-Tetrahydrochinolin-4-yl)-ethyl]-4-(3,4-dimethoxyphenyl)-piperidin, sowie deren Salze

4. Verfahren zur Herstellung von Piperidin- und Piperazin-Derivaten der Formel I gemäß Anspruch 1, dadurch gekennzeichnet, daß man eine Verbindung der Formel II 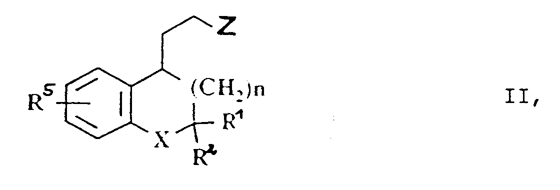 worin
Z Cl, Br, OH oder eine reaktionsfähige, funktionell abgewandelte OH-Gruppe bedeutet und
R¹, R², R⁵, X und n die angegebenen Bedeutungen haben,
mit einer Verbindung der Formel III 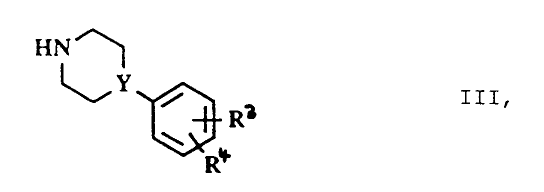 worin R³, R⁴ sowie Y die angegebenen Bedeutungen haben,
umsetzt,
oder daß man eine Verbindung, die an sich der Formel I entspricht, aber anstelle einer oder mehrerer CH₂-Gruppen eine oder mehrere reduzierbare Gruppen besitzt, durch Reduktion in eine Verbindung der Formel I überführt und/oder daß man in einer Verbindung der Formel I einen oder mehrere der Reste R³, R⁴ und/oder R⁵ bzw. X, sofern X = NH ist, in andere Reste R³, R⁴ und/oder R⁵ bzw. X umwandelt und/oder daß man eine basische Verbindung der Formel I durch Behandeln mit einer Säure in eines ihrer physiologisch unbedenklichen Säureadditionssalze umwandelt.

5. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

6. Pharmazeutische Zubereitung, gekennzeichnet durch einen Gehalt an mindestens einer Verbindung der Formel I und/oder einem ihrer physiologisch unbedenklichen Salze.

7. Verwendung von Verbindungen der Formel I nach Anspruch 1 oder von deren physiologisch unbedenklichen Salzen zur Herstellung eines Arzneimittels.

8. Verwendung von Verbindungen der Formel I nach Anspruch 1 oder von deren physiologisch unbedenklichen Salzen bei der Bekämpfung von Krankheiten.
